(19) Europäisches Patentamt
European Patent Office
Office européen des brevets

(11) Veröffentlichungsnummer: **0 093 693**
**B1**

(12) **EUROPÄISCHE PATENTSCHRIFT**

(45) Veröffentlichungstag der Patentschrift: **31.05.89**

(51) Int. Cl.⁴: **C 07 D 401/14,** C 08 K 5/34

(21) Anmeldenummer: **83810175.6**

(22) Anmeldetag: **28.04.83**

# (54) Verfahren zur Herstellung von Polyaminotriazinen.

(30) Priorität: **04.05.82 CH 2732/82**

(43) Veröffentlichungstag der Anmeldung: **09.11.83 Patentblatt 83/45**

(45) Bekanntmachung des Hinweises auf die Patenterteilung: **31.05.89 Patentblatt 89/22**

(84) Benannte Vertragsstaaten: **CH DE FR GB IT LI**

(56) Entgegenhaltungen:
**EP-A-0 044 499**
**EP-A-0 063 775**
**EP-A-0 065 169**
**DE-A-2 933 078**

**S-Traizines and Derivatives, E.M. Smolin, L. Rapoport, Intersciences Publ., 1959, p. 53**

(73) Patentinhaber: **CIBA- GEIGY AG, Klybeckstrasse 141, CH- 4002 Basel (CH)**

(72) Erfinder: **Orban, Ivan, Dr., Lehenmattstrasse 216, CH- 4052 Basel (CH)**
Erfinder: **Troxler, Eduard, Dr., St. Albanring 220, CH- 4052 Basel (CH)**

**EP 0 093 693 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung ist ein verbessertes Verfahren zur Herstellung von Polyaminotriazinen durch Polykondensation von Dichlortriazinen mit sekundären Diaminen, welche am Stickstoff durch 2,2,6,6-Tetramethylpiperidin-4-ylreste substituiert sind.

In der DE-OS-2 636 144 werden Verbindungen der Formel

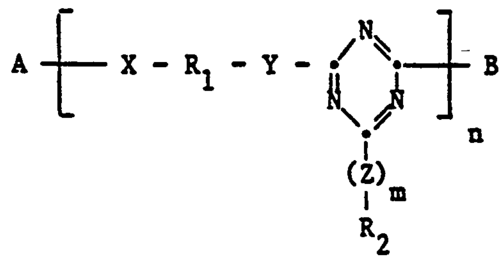

beschrieben, worin X, Y und Z -O-, -NH- oder -$NR_3$- sind, $R_1$ ein zweiwertiger organischer Rest und $R_2$ und $R_3$ einwertige Reste sind und mindestens einer von $R_2$ oder $R_3$ ein Polyalkylpiperidinrest ist, m null oder 1 ist, n eine Zahl von 2 - 200 ist und A und B Endgruppen sind. Solche Verbindungen sind ausgezeichnete Lichtschutzmittel, insbesondere für organische Polymere, und haben technische Verwendung zum Stabilisieren von Kunststoffen gefunden. Gemäss der DE-OS-2 636 144 geschieht die Herstellung dieser Polyaminotriazine durch Polykondensation eines Dichlortriazines der Formel

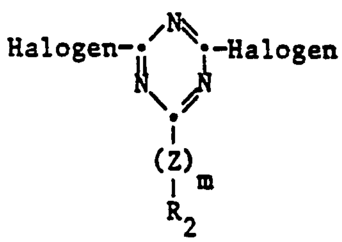

oder eines Cyanursäurehalogenides in einem inerten Lösungsmittel bei einer Temperatur von -10$^{c}$ bis zur Siedetemperatur des Lösungsmittels in Gegenwart einer organischen oder anorganischen Base.

In den Ausführungsbeispielen wird die Reaktion vorwiegend in siedendem Toluol ausgeführt, wobei als Base wasserfreies NaOH verwendet wird. Die Aufarbeitung geschieht durch Abfiltrieren des gebildeten NaCl und Verdampfen des Lösungsmittels. Dieses Verfahren hat den Nachteil, dass die Reaktion nicht vollständig abläuft. Ein zweiter Nachteil ist, dass sich schwerlösliche Nebenprodukte bilden, die vor allem aus cyclischen Produkten bestehen. Diese sind wegen ihrer Schwerlöslichkeit als Stabilisatoren nicht erwünscht und müssen vom linearen Polykondensat durch Filtration abgetrennt werden. Neben dem Ausbeuteverlust durch Bildung dieser Nebenprodukte hat dies den Nachteil, dass diese sich sehr schlecht abfiltrieren lassen, da sie in sehr feinpulvriger Form anfallen und dadurch die Filterporen verstopfen. Nach Abtrennung dieser unerwünschten Nebenprodukte beträgt daher die Ausbeute beim angegebenen Verfahren nur etwa 70 - 75 %. Ein dritter Nachteil des Verfahrens ist, dass nur Produkte mit einem relativ niedrigen Polykondensationsgrad $n < 6$ erhältlich sind. Im allgemeinen ist für die Verwendung als Stabilisator ein niedriges n erwünscht, für spezielle Verwendungszwecke wären aber Produkte mit höherem Molekulargewicht erwünscht.

Will man die Ausbeute des bekannten Verfahrens durch die Verwendung eines Überschusses an NaOH verbessern, so tritt verstärkte Bildung der unlöslichen Nebenprodukte sowie eine teilweise Hydrolyse des Halogens am Triazinring ein, so dass keine wesentliche Erhöhung der Ausbeute an linearen Polyaminotriazinen erzielt wird.

In der DE-OS-2 933 078 werden ganz ähnliche Polyaminotriazine beschrieben, die ebenfalls durch Polykondensation in siedendem Toluol oder Xylol unter Zusatz von wasserfreiem NaOH hergestellt werden. Hierdurch werden auch dort nur Produkte von sehr niedrigem Molekulargewicht in ungenügender Ausbeute erhalten.

Es wurde nunmehr gefunden, dass man die geschilderten Nachteile weitgehend vermeiden kann, wenn man bei höheren Temperaturen arbeitet und die Reaktion zweiphasig ausführt. Hierzu verwendet man ein mit Wasser nicht mischbares Lösungsmittel, in dem die Ausgangsmaterialien und die Endprodukte löslich sind, und verwendet als Base eine konzentrierte wässrige Lösung einer anorganischen Base. Damit man bei höheren Temperaturen arbeiten kann, führt man die Reaktion unter Druck aus.

Gegenstand der Erfindung ist somit ein Verfahren zur Herstellung von Verbindungen der Formel I

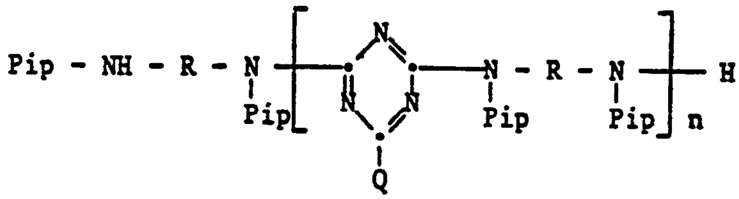

(I),

worin n 2 - 20 ist,

Pip einen Rest der Formel

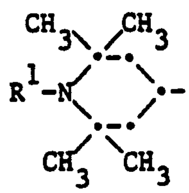

darstellt, worin $R^1$ Wasserstoff, $C_1$-$C_{12}$-Alkyl, $C_3$-$C_8$-Alkenyl, $C_7$-$C_{11}$-Phenylalkyl, $C_2$-$C_8$-Alkanoyl oder $C_3$-$C_5$-Alkenoyl ist,

R $C_2$ - $C_{12}$-Alkylen, das durch -O- oder -$NR^2$- unterbrochen sein kann, worin $R^2$ Wasserstoff, $C_1$ - $C_{12}$-Alkyl, $C_3$ - $C_{12}$-Alkoxyalkyl, Cycloalkyl oder ein Rest Pip ist, oder R einen zweiwertigen cycloaliphatischen $C_6$-$C_{15}$-Rest bedeutet,

Q ein Rest der Formel -$OR^3$, -$NHR^4$ oder -$NR^4R^5$ ist, worin $R^3$ $C_1$ - $C_{12}$-Alkyl $C_3$ - $C_{12}$-Alkoxyalkyl, Cyclohexyl, Benzyl, Phenyl, Tolyl oder Pip bedeutet, $R^4$ $C_1$ - $C_{12}$-Alkyl, $C_3$ - $C_{12}$-Alkoxyalkyl, $C_4$ - $C_{12}$-Dialkylaminoalkyl, Allyl, Benzyl, Cyclohexyl, Phenyl, Tolyl oder ein Rest Pip bedeutet und $R^5$ $C_1$ - $C_{12}$ -Alkyl $C_3$ - $C_{12}$-Alkoxyalkyl oder Cyclohexyl bedeutet, oder $R^4$ und $R^5$ zusammen mit dem N-Atom, an das sie gebunden sind, einen 5- oder 6-gliedrigen heterocyclischen Ring bilden, durch Polykondensation von 1 Mol eines Dichlortriazins der Formel II

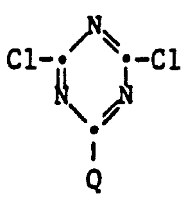

(II)

mit 1,00 bis 1,20 Mol eines Diamines der Formel III

Pip - NH - R - NH - Pip (III)

in einem inerten organischen Lösungsmittel unter Zusatz einer Base, dadurch gekennzeichnet, dass man als Base eine wässrige Lösung einer anorganischen Base verwendet, dass man ein mit Wasser nicht mischbares Lösungsmittel verwendet, und die Umsetzung bei 140 - 220°C unter Druck ausführt.

R kann ein unverzweigter oder verzweigter Alkylenrest sein, der durch -O- oder -$NR^2$- unterbrochen sein kann. Beispiele hierfür sind 1,2-Ethylen, Tri-, Tetra-, Penta-, Hexa-, Octa-, Deca- oder Dodecamethylen, 2,2,4- oder 2,4,4-Trimethylhexamethylen, 4-Oxaheptylen-1,7, 4,7-Dioxadecylen-1,10, 4-(Methylaza)-heptylen-1,7, 4-(Cyclohexyl-aza)-heptylen-1,7 oder 4-(1,2,2,6,6-Pentamethyl-4-piperidyl-aza)-heptylen-1,7. R kann auch ein cycloaliphatischer Rest sein wie z. B. 1,3- oder 1,4-Cyclohexylen, Dicyclohexylmethan-4,4'-diyl oder ein Rest der Formel

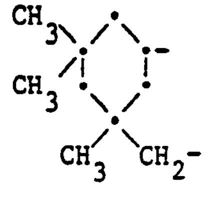

.

$R^1$, $R^2$, $R^3$, $R^4$ und $R^5$ als Alkyl können z. B. Methyl, Ethyl, Propyl, Isopropyl, n-Butyl, tert.Butyl, Isoamyl, n-Hexyl, 2-Ethylhexyl, n-Octyl, ditert.-Octyl, n-Decyl oder n-Dodecyl sein. $R^3$ $R^4$ und $R^5$ als Alkoxyalkyl können z. B. 2-Methoxyethyl, 2-Ethoxy-ethyl, 3-Ethoxypropyl, 3-Butoxypropyl oder 3-Isopropoxypropyl sein. $R^4$ als Dialkylaminoalkyl kann z. B. 2-Dimethylaminoethyl, 3-Dimethylaminopropyl, 3-Diethylaminopropyl oder 3-Dibutylaminopropyl sein.

$R^1$ als Alkenyl kann z. B. Allyl, Methallyl oder Dimethylallyl sein.

$R^1$ als Phenylalkyl kann z. B. Benzyl, Phenylethyl oder Phenylpropyl sein.

$R^1$ als Alkanoyl kann z. B. Acetyl, Propionyl, Butyroyl, Hexanoyl oder Octanoyl sein. $R^1$ als Alkenoyl kann z. B. Acryloyl, Methacryloyl oder Crotonoyl sein.

Dieses Verfahren ermöglicht einen praktisch quantitativen Umsatz und eine hohe Ausbeute an linearen Polytriazinen der Formel I. Dabei wird nicht nur der Umsatz erhöht sondern auch die Bildung der schwerlöslichen Nebenprodukte zurückgedrängt und diese fallen in einer leichter filtrierbaren, gröberen Form an. Dadurch wird ein schwerwiegendes technisches Problem gelöst.

Weiterhin ist es aufgrund des hohen Umsatzes möglich, Produkte mit höherem Polykondensationsgrad n zu erhalten. Wenn man jedoch Produkte mit niedrigem n anstrebt, so kann man dies auch beim vorliegenden Verfahren in bekannter Weise durch Verwendung eines entsprechenden Überschusses des Diamines III erreichen.

Ein kleiner Überschuss an Diamin ist vorteilhaft. Je nach dem gewünschten Molekulargewicht bzw. Polykondensationsgrad verwendet man 0 - 20 Mol-%, vorzugsweise 2 - 5 Mol-%, Überschuss an III. Man erreicht dadurch Polykondensationsgrade n von 2 - 20, vorzugsweise 4 - 10.

Überraschend ist, dass bei Verwendung einer wässrigen Base, die Bildung von schwerlöslichen Nebenprodukten zurückgedrängt und die Ausbeute erhöht wird. Es war gemäss Literatur (E. M. Smolin, L. Rapoport, s-Triazines and Derivatives, Interscience Publ., 1959 Seite 53) zu erwarten, dass unter wässrigalkalischen Bedingungen, im Temperaturbereich über 140° C, die Cl-Atome verseift werden.

Das Verfahren ist mit einer Vielzahl von Dichlortriazinen der Formel II und Diaminen der Formel III durchführbar. Bevorzugt verwendet man jedoch solche Dichlortriazine der Formel II, worin Q ein Rest -NHR ist und solche Diamine der Formel III, worin R ein unverzweigter oder verzweigter $C_4$ - $C_{12}$-Alkylenrest ist und $R^1$ Wasserstoff oder Methyl ist. Besonders bevorzugt verwendet man Dichlortriazine der Formel II, worin Q der Rest -NH-$C_8$-$C_{12}$-Alkyl ist, und solche Diamine der Formel III, worin R Hexamethylen und $R^1$ Wasserstoff oder Methyl sind.

Die Dichlortriazine der Formel II können aus Cyanursäuretrichlorid und einer monofunktionellen Verbindung QH hergestellt werden, wie dies in der DE-OS-2 636 144 oder in der DE-OS-2 933 078 beschrieben ist. Die hierbei erhaltene Reaktionslösung kann - ohne Isolierung der Verbindung II - für das vorliegende Verfahren verwendet werden. Vorzugsweise isoliert man jedoch die Verbindung II, ehe man sie weiter umsetzt.

Die Diamine der Formel III können durch hydrierende Aminierung von Triacetonamin hergestellt werden, wie dies in der DE-OS-2 611 208 beschrieben ist.

Als organisches Lösungsmittel kommen vor allem Kohlenwasserstoffe in Frage wie z. B. Toluol, Xylol, Mesitylen, Tetralin, Dekalin oder höhere Alkylbenzole wie z. B. Nonylbenzol oder Dodecylbenzol oder Gemische solcher Alkylbenzole.

Als anorganische Basen kommen vor allem solche in Frage, die eine hohe Löslichkeit in Wasser besitzen wie z. B. Alkalihydroxide oder Alkalicarbonate. Bevorzugt verwendet man Natrium- oder Kaliumhydroxid. Man arbeitet vorzugsweise mit 30 - 60 %-iger, insbesondere etwa 50 %-igen, Lösungen der anorganischen Base.

Die verwendeten Temperaturen sind höher als bisher beschrieben wurde, vorzugsweise arbeitet man bei 170 - 190° C. Man arbeitet dabei in einem geschlossenen Gefäss, so dass durch den bei der Reaktionstemperatur herrschenden Dampfdruck des Lösungsmittels und des Wassers ein Überdruck entsteht. Dieser beträgt etwa 0,5 - 1 Mpa, so dass für die Durchführung der Reaktion keine dickwandigen Autoklaven nötig sind. Vorzugsweise führt man die Reaktion in einem drucksicheren Rührkessel aus. Das Rühren soll nicht intensiv sein, eine turbulente Mischung der beiden Phasen ist zu vermeiden. Zu empfehlen ist die Verwendung eines Ankerrührers mit einer Rührgeschwindigkeit von 40 - 45 Umdrehungen pro Minute.

Vorzugsweise legt man die gesamte Lösung des Diamines der Formel III und der Base vor, erwärmt die Lösung auf die gewünschte Reaktionstemperatur und dosiert die Lösung der Triazinkomponente der Formel II langsam unter Rühren zu. Man kann jedoch auch alle Reaktionskomponenten vorlegen und das Gemisch auf die gewünschte Reaktionstemperatur heizen. In diesem Fall wird der Polykondensationsgrad n etwas niedriger.

Am Ende der Reaktion trennt man die wässrige Phase ab, trocknet die organische Phase - vorzugsweise durch azeotrope Destillation - und filtriert die Lösung. Die Filtration kann beschleunigt werden durch Zusatz eines Filtrierhilfsmittels, z. B. eines Kieselgurs oder einer Fullererde. Nach Verdampfen des Lösungsmittels hinterbleibt das Polyaminotriazin als bei Raumtemperatur feste Masse, die je nach Bedarf zerkleinert werden kann. Die folgenden Ausführungsbeispiele illustrieren das Verfahren näher, ohne es auf die Verfahrensweise der Beispiele zu begrenzen.

**Beispiel 1**

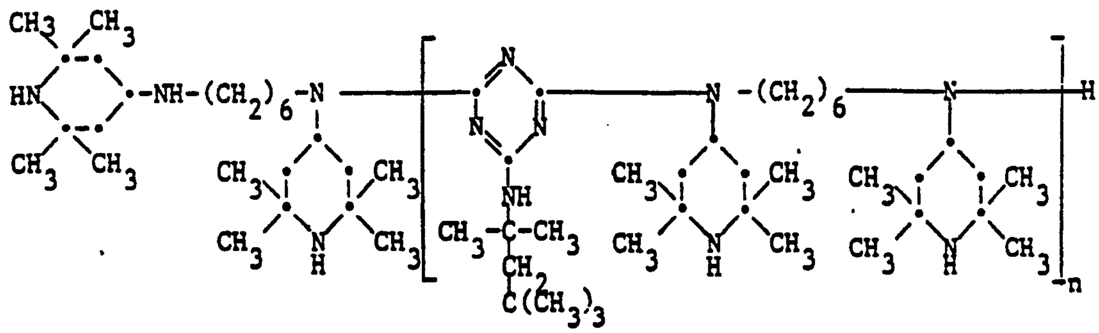

In einem 2,5 l-Niederdruckautoklaven werden bei 180°C 413,7 g N,N'-Bis-(2,2,6,6-tetramethyl-4-piperidyl)-hexamethylendiamin (1,05 Mol) und 175 g 50 %-ige wässrige Natronlauge vorgelegt. Dazu wird unter Rühren eine Lösung von 276,5 g tert.-Octylamino-dichlor-s-triazin (1,0 Mol) (hergestellt aus Cyanurchlorid und tert.Octylamin) in 450 g Xylol wie folgt zudosiert: 85 % der Xylollösung innerhalb 3 h, dann die restlichen 15 % in 4 h. Es stellt sich ein maximaler Druck von 0,7 Mpa ein. Nach einer Haltezeit von weiteren 5 h bei 185°C wird das Reaktionsgemisch auf 80 - 90°C gekühlt und einmal mit Wasser gewaschen. Nach Abtrennen der wässrigen Phase wird die Xylol-Phase azeotrop entwässert und nach Zugabe von Filtrierhilfsmittel Celite 545 filtriert. Das klare Filtrat wird im Rotationsverdampfer bei 200°C eingedampft, die zurückgebliebene Schmelze abgekühlt und das erhaltene Festharz zerkleinert.
Ausbeute: 573,5 g = 93 % d. Th.
Durch Gelpermeationsanalyse und Dampfdruckosmometrie wurde ein mittleres Molekulargewicht $\bar{M}_n$ (Zahlenmittel) von 4900 gefunden. Dies entspricht einem Polykondensationsgrad n = 7,5.

**Beispiel 2**

Analog Beispiel 1 werden 403,8 g (1,025 Mol) N,N'-Bis-(2,2,6,6-tetramethyl-4-piperidyl)-hexamethylendiamin mit den in Beispiel 1 angegebenen Mengen an Dichlortriazin und Natronlauge umgesetzt. Unter denselben Bedingungen der Aufarbeitung erhält man 583 g = 96 % d. Th. $\bar{M}_n$ = 6300, entsprechend n = 9,9.

**Beispiel 3**

Es wird wie im Beispiel 1 verfahren, jedoch werden 423,6 g (1,075 Mol) des Diamines verwendet.
Ausbeute: 5914 g = 94,3 % d.Th. $\bar{M}_n$ = 3660, entsprechend n = 5;5.

**Patentansprüche**

1. Verfahren zur Herstellung von Verbindungen der Formel I

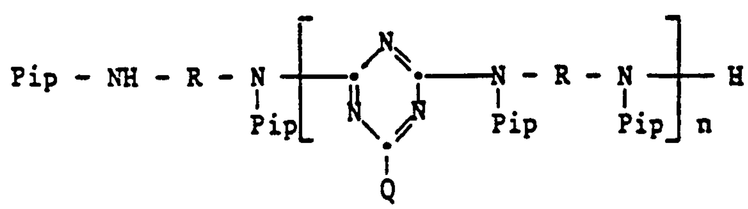 (I),

worin n 2 - 20 ist, Pip einen Rest der Formel

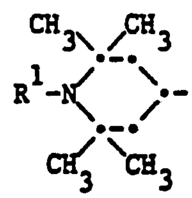

darstellt, worin $R^1$ Wasserstoff, $C_1$ - $C_{12}$-Alkyl, $C_3$ - $C_8$-Alkenyl, $C_7$-$C_{11}$-Phenylalkyl, $C_2$-$C_8$-Alkanoyl oder $C_3$-$C_5$-Alkenoyl ist,

R $C_2$-$C_{12}$-Alkylen, das durch -O- oder -$NR^2$- unterbrochen sein kann, worin $R^2$ Wasserstoff, $C_1$ - $C_{12}$-Alkyl, $C_3$ - $C_{12}$-Alkoxyalkyl, Cycloalkyl, oder ein Rest Pip ist, oder einen zweiwertigen cycloaliphatischen $C_6$ - $C_{15}$-Rest bedeutet,

Q ein Rest der Formel -$OR^3$, -$NHR^4$ oder -$NR^4R^5$ ist, worin $R^3$ $C_1$ - $C_{12}$-Alkyl, $C_3$ - $C_{12}$-Alkoxyalkyl, Cyclohexyl, Benzyl, Phenyl, Tolyl oder Pip bedeutet, $R^4$ $C_1$ - $C_{12}$-Alkyl, $C_3$ - $C_{12}$-Alkoxyalkyl, $C_4$ - $C_{12}$-Dialkylaminoalkyl, Allyl, Benzyl, Cyclohexyl, Phenyl, Tolyl oder einen Rest Pip bedeutet und $R^5$ $C_1$ - $C_{12}$-Alkyl, $C_3$ - $C_{12}$-Alkoxyalkyl oder Cyclohexyl bedeutet oder $R^4$ und $R^5$ zusammen mit dem N-Atom, an das sie gebunden sind, einen 5- oder 6-gliedrigen heterocyclischen Ring bilden, durch Polykondensation von 1 Mol eines Dichlortriazins der Formel II

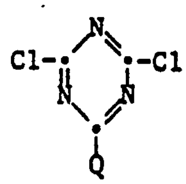

(II)

mit 1,00 bis 1,20 Mol eines Diamins der Formel III

Pip - NH - R - NH - Pip (III)

in einem inerten organischen Lösungsmittel unter Zusatz einer Base, dadurch gekennzeichnet, dass man als Base eine wässrige Lösung einer anorganischen Base verwendet, dass man ein mit Wasser nicht mischbares Lösungsmittel verwendet und die Umsetzung bei 140 - 220° C unter Druck ausführt.

2. Verfahren gemäss Anspruch 1 zur Herstellung von Verbindungen der Formel I, worin $R^1$ Wasserstoff oder Methyl ist, R ein unverzweigter oder verzweigter $C_4$ - $C_{12}$-Alkylenrest ist und Q ein Rest -$NHR^4$ ist.

3. Verfahren gemäss Anspruch 2 zur Herstellung von Verbindungen der Formel I, worin R Hexamethylen und $R^4$ ein $C_6$ - $C_{12}$-Alkylrest sind.

4. Verfahren gemäss Anspruch 1, dadurch gekennzeichnet, dass man als Base eine konzentrierte wässrige Lösung von NaOH oder KOH verwendet.

5. Verfahren gemäss Anspruch 1, dadurch gekennzeichnet, dass man das Diamin III in einem Überschuss von 1 - 10 Mol-% verwendet.

6. Verfahren gemäss Anspruch 1, dadurch gekennzeichnet, dass man das Reaktionsgemisch so langsam rührt, dass es nicht zu einer turbulenten Vermischung der beiden Phasen kommt.

7. Verfahren gemäss Anspruch 1, dadurch gekennzeichnet, dass man die Umsetzung bei 180 - 190°C durchführt.

8. Verfahren gemäss Anspruch 1, dadurch gekennzeichnet, dass man die Triazinkomponente der Formel II bei Reaktionstemperatur zur Lösung des Diamines der Formel III langsam zugibt.

**Claims**

1. A process for the preparation of a compound of the formula I

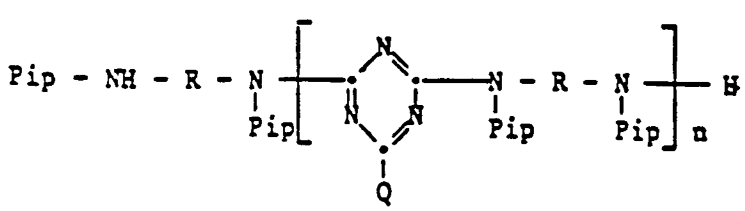

(I),

in which n is a number from 2 to 20, Pip is a radical of the formula

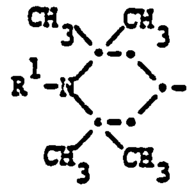

in which $R^1$ is hydrogen, $C_1$ - $C_{12}$alkyl, $C_3$ - $C_8$alkenyl, $C_7$ - $C_{11}$phenylalkyl, $C_2$ - $C_8$alkanoyl or $C_3$ - $C_5$alkenoyl, R is $C_2$ - $C_{12}$alkylene, which can be interrupted by -O- or $-NR^2-$, in which $R^2$ is hydrogen, $C_1$ - $C_{12}$alkyl, $C_3$ - $C_{12}$-alkoxyalkyl, cycloalkyl or Pip a radical, or a divalent cycloaliphatic $C_6$ - $C_{15}$radical, and Q is a radical of the formula $-OR^3$, $-NHR^4$ or $-NR^4R^5$, in which $R^3$ is $C_1$ - $C_{12}$alkyl, $C_3$ - $C_{12}$alkoxyalkyl, cyclohexyl, benzyl, phenyl, tolyl or Pip, $R^4$ is $C_1$ - $C_{12}$alkyl, $C_3$ - $C_{12}$alkoxyalkyl, C4 - $C_{12}$dialkylaminoalkyl, allyl, benzyl, cyclohexyl, phenyl, tolyl or Pip a radical and $R^5$ is $C_1$ - $C_{12}$alkyl, $C_3$ - $C_{12}$alkoxyalkyl or cyclohexyl, or $R^4$ and $R^5$, together with the N-atom to which they are bonded, form a 5- or 8-membered heterocyclic ring, by polycondensation of 1 mol of a dichlorotriazine of the formula II

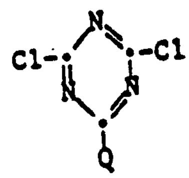

(II)

with 1.00 to 1.20 moles of a diamine of the formula III
Pip - NH - R - NH - Pip (III)
in an inert organic solvent with addition of a base, wherein an aqueous solution of an inorganic base is used as the base, a water-immiscible solvent is used, and the reaction is carried out at 140 - 220°C under pressure.

2. A process according to claim 1 for the preparation of a compound of the formula I in which $R^1$ is hydrogen or methyl, R is a straight-chain or branched $C_4$-$C_{12}$alkylene radical and Q is a radical $-NHR^4$.

3. A process according to claim 2 for the preparation of compounds of the formula I in which R is hexamethylene and $R^4$ is a $C_6$ - $C_{12}$alkyl radical.

4. A process according to claim 1, wherein a concentrated aqueous solution of NaOH or KOH is used as the base.

5. A process according to claim 1, wherein the diamine III is used in an excess of 1 - 10 mol %.

6. A process according to claim 1, wherein the reaction mixture is stirred so slowly that no turbulent mixing of the two phases occurs.

7. A process according to claim 1, wherein the reaction is carried out at 180 - 190°C.

8. A process according to claim 1, wherein the triazine component of the formula II is slowly added, at the reaction temperature, to the solution of the diamine of the formula III.

## Revendications

1. Procédé pour préparer des composés répondant à la formule I:

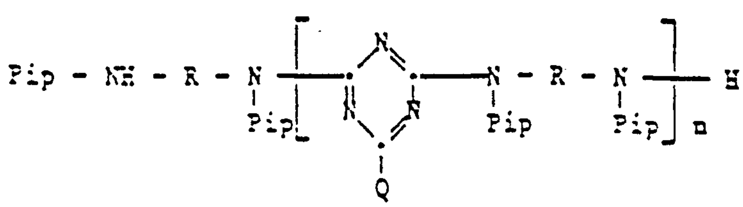

(I)

dans laquelle :
n désigne un nombre de 2 à 20,
Pip représente un radical de formule:

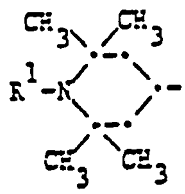

où $R^1$ désigne l'hydrogène, un alkyle en $C_1$ - $C_{12}$, un alcényle en $C_3$ - $C_8$, un phénylalkyle en $C_7$ - $C_{11}$, un alcanoyle en $C_2$ - $C_8$ ou un alcénoyle en $C_3$ - $C_5$,

R représente un alkylène en $C_2$ - $C_{12}$ qui peut être interrompu par -O- ou -$NR^2$-, le symbole $R^2$ désignant l'hydrogène, un alkyle en $C_1$ - $C_{12}$, un alcoxyalkyle en $C_3$ - $C_{12}$, un cycloalkyle ou un radical Pip,

ou

R représente un radical cycloaliphatique bivalent en $C_6$ - $C_{15}$,

Q représente un radical -$OR^3$, -$NHR^4$ ou -$NR^4R^5$, où $R^3$ représente un alkyle en $C_1$ - $C_2$, un alcoxyalkyle en $C_3$ - $C_{12}$, un cyclohexyle, un benzyle, un phényle, un tolyle ou Pip, $R^4$ représente un alkyle en $C_1$ - $C_{12}$, un alcoxyalkyle en $C_3$ - $C_{12}$,+ un dialkylaminoalkyle en $C_4$ - $C_{12}$, un allyle, un benzyle, un cyclohexyle, un phényle, un tolyle ou un radical Pip et $R^5$ représente un alkyle en $C_1$ - $C_{12}$, un alcoxyalkyle en $C_3$ - $C_{12}$ ou un cyclohexyle, ou $R^4$ et $R^5$ forment ensemble, et avec l'atome d'azote auquel ils sont liés, un radical hétérocyclique à 5 ou 6 maillons,

par polycondensation de 1 mol d'une dichlorotriazine de formule II:

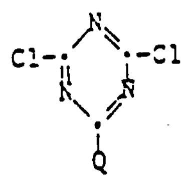

(II)

avec de 1,00 à 1.20 mol d'une diamine de formule III:

Pip - NH - R - NH - Pip (III),

dans un solvant organique inerte, en présence d'une base, procédé caractérisé en ce qu'on utilise, comme base, une solution aqueuse d'une base minérale, en ce qu'on utilise un solvant non miscible à l'eau et en ce qu'on effectue la réaction à une température de 140 à 220°C sous pression.

2. Procédé selon la revendication 1 pour préparer des composés de formule I dans lesquels $R^1$ represente l'hydrogène ou un méthyle, R représente un radical alkylène en $C_4$ - $C_{12}$ linéaire ou ramifié et Q représente un radical -$NHR^4$.

3. Procédé selon la revendication 2 pour préparer des composés de formule I dans lesquels R représente un radical hexaméthylène et $R^4$ un radical alkyle en $C_6$ - $C_{12}$.

4. Procédé selon la revendication 1 caractérisé en ce qu'on utilise, comme base, une solution aqueuse concentrée de NaOH ou de KOH.

5. Procédé selon la revendication 1 caractérisé en ce qu on utilise la diamine (III) en un excès le 1 à 10 % en moles.

6. Procédé selon la revendication 1 caractérisé en ce qu'on agite le mélange réactionnel suffisamment lentement pour qu'il ne se produise pas un mélangeage turbulent des deux phases.

7. Procédé selon la revendication 1 caractérisé en ce qu'on effectue la réaction à une température de 180 à 190°C.

8. Procédé selon la revendication 1 caractérisé en ce qu'on ajoute lentement la composante triazinique de formule II, à la température réactionnelle, à la solution de la diamine de formule III.